# EUROPÄISCHE PATENTSCHRIFT

(11) **EP 1 115 713 B1**
(45) Veröffentlichungstag und Bekanntmachung des Hinweises auf die Patenterteilung: **06.05.2004**
(21) Anmeldenummer: 99969411.0
(22) Anmeldetag: 20.09.1999
(51) Int. Cl.: C07D 301/10, F22B 1/18

(54) **VERFAHREN ZUR HERSTELLUNG VON ETHYLENOXID DURCH DIREKTOXIDATION VON ETHYLEN MIT LUFT ODER SAUERSTOFF**
METHOD FOR PRODUCING ETHYLENE OXIDE BY DIRECTLY OXIDIZING ETHYLENE WITH AIR OR OXYGEN
PROCEDE DE PRODUCTION D'OXYDE ETHYLENIQUE PAR OXYDATION DIRECTE D'ETHYLENE AVEC DE L'AIR OU DE L'OXYGENE

(30) Priorität: 23.09.1998 DE 19843654
(43) Veröffentlichungstag der Anmeldung: 18.07.2001
(73) Patentinhaber: BASF AKTIENGESELLSCHAFT, 67056 Ludwigshafen (DE)
(72) Erfinder: THEIS, Gerhard, D-67133 Maxdorf (DE); VANSANT, Frans, B-2920 Kalmthout (BE)
(74) Vertreter: Isenbruck, Günter, Dr.
(86) Internationale Anmeldenummer: PCT/EP1999/006941
(87) Internationale Veröffentlichungsnummer: WO 2000/017177

(56) Entgegenhaltungen:
- EP-A- 0 532 325
- DE-A- 3 935 030
- US-A- 3 552 122
- DATABASE WPI Section Ch, Week 197517 Derwent Publications Ltd., London, GB; Class E13, AN 1975-28267W XP002128832 & JP 50 007574 B (JAPAN CATALYTIC CHEM IND CO), 27. März 1975 (1975-03-27)

## Beschreibung

Die Erfindung betrifft ein Verfahren zur Herstellung von Ethylenoxid (im folgenden mit EO bezeichnet) durch Direktoxidation von Ethylen sowie ein Verfahren, wonach aus EO durch Hydrolyse, Druckentwässerung, Vakuumentwässerung und anschließender Reindestillation Glykol gewonnen wird.

Zur Zeit wird EO großtechnisch durch Direktoxidation von Ethylen mit Luft oder Sauerstoff an Silberkatalyatoren hergestellt. Die Reaktion ist stark exotherm (Gesamtwärmetönung 225 bis 400 KJ pro Mol Ethylen); daher wird zur Abführung der überschüssigen Reaktionswärme üblicherweise in Rohrbündelreaktoren gearbeitet, wobei das Reaktionsgemisch durch die Rohre geführt wird und zwischen den Rohren als Wärmeträger eine siedende Flüssigkeit, beispielsweise Kerosin oder Tetralin, neuerdings vielfach Wasser; zirkuliert. Die vorliegende Erfindung betrifft Verfahren, wonach als Wärmeträger Wasser verwendet wird.

Derartige Verfahren sind beispielsweise in Ullmanns Encyclopedia of Industrial Chemistry, Fifth Edition, Vol. A 10, pages 117ff beschrieben. Danach werden Ethylen und Sauerstoff in einem Kreisgasstrom vorgelegt, der neben den Reaktanden Inertgase und das Nebenprodukt der Ethylentotaloxidation, Kohlendioxid, enthält.

Der in der Ethylen-Direktoxidation anfallende Wasserdampf wird im bekannten Verfahren in der Regel über ein Ventil auf den Druck eines Dampfnetzes entspannt.

Dabei wird der Energiegehalt des Wasserdampfes aus der Entspannung nicht genutzt.

Ein bedeutender Anteil an Ethylenoxid (EO) aus der weltweiten Produktion wird, mit steigender Tendenz, zu Monoethylenglykol weiterverarbeitet. Zur Verbesserung der Selektivität der EO-Hydrolyse wird der Hydrolysereaktor mit einem großen Wasserüberschuß (Gewichtsverhältnis Wasser: EO bis 15:1) betrieben. Dadurch kann der Anteil an höheren Glykolen, insbesondere an Diethylenglykol, Triethylenglykol usw. zurückgedrängt werden. Der Hydrolysereaktor wird üblicherweise bei Temperaturen von 120°C bis 250°C und Drücken von 30 - 40 bar betrieben. Das Hydrolyseprodukt wird zunächst entwässert, auf einen Restwassergehalt von 100 - 200 ppm und anschließend in die verschiedenen Glykole in reiner Form aufgetrennt.

Die Entwässerung erfolgt in der Regel in einer Kaskade von druckgestaffelten Kolonnen, mit abnehmendem Druck. Aus Gründen der Wärmeintegration wird in der Regel nur der Sumpfverdampfer der ersten Druckkolonne mit Frischdampf beheizt, alle weiteren Druckkolonnen dagegen mit den Brüden der jeweils voranstehenden Kolonne. Je nach Wassergehalt des Hydrolysereaktoraustrags und des Druck/Temperaturniveaus des im Sumpfverdampfer der ersten Kolonne eingesetzten Fremddampfs besteht die Druckentwässerungskaskade aus 2 bis 7 Kolonnen. An die Druckentwässerung schließt sich eine Vakuumentwässerung an. Die entwässerte, Glykole enthaltende Lösung wird in mehreren Kolonnen in die Reinstoffe Monoethylenglykol, Di- und Triethylenglykol zerlegt.

Es ist Aufgabe der Erfindung, den bei der Direktoxidation von Ethylenoxid unter Verwendung von Wasser als Wärmeüberträger anfallenden Wasserdampf energetisch optimal zu nutzen und die Wirtschaftlichkeit des Verfahrens zur Herstellung von EO und/oder Monoethylenglykol zu verbessern.

Die Lösung geht aus von einem Verfahren zur Herstellung von EO durch Direktoxidation von Ethylen mit Luft oder Sauerstoff unter Verwendung von Wasser als Wärmeüberträger, wobei Wasserdampf entsteht, der anschließend entspannt wird. Die Erfindung ist dadurch gekennzeichnet, daß die Entspannung des Wasserdampfes in einer oder mehreren Gegendruck-Dampfturbine(n) erfolgt.

Dampfturbinen bezeichnen in bekannter Weise Wärmekraftmaschinen mit rotierenden Laufteilen, in denen das Druckgefälle stetig strömenden Dampfes in einer oder mehreren Stufen in mechanische Arbeit umgewandelt wird. Je nach Art der Dampfabführung werden verschiedene Typen von Dampfturbinen unterschieden; bei sogenannten Gegendruck-Dampfturbinen wird die Abdampfenergie noch für andere Zwecke, meist zur Heizung, ausgenutzt.

Für den Einsatz im vorliegenden Verfahren kann prinzipiell jede Gegendruck-Dampfturbine verwendet werden.

Dampfturbinen werden üblicherweise mit Dampfzuführung unter konstanten Bedingungen betrieben. Demgegenüber wird gemäß der Erfindung eine Dampfturbine mit kontinuierlich zunehmender Dampfmenge und ansteigendem Dampfdruck betrieben. Für die Wirtschaftlichkeit dieser Lösung sind die Bedingungen (Dampfmenge, Druck) beim zeitlichen Mittelwert ausschlaggebend.

Die in der Ethylenoxidation eingesetzten technischen Katalysatoren, die im allgemeinen bis zu 15 Gew.-% Silber in der Form einer feinteiligen Schicht auf einem Träger enthalten, verlieren mit zunehmender Betriebsdauer an Aktivität, die Selektivität der Partialoxidation von Ethylenoxid nimmt ab. Um mit fortschreitender Betriebsdauer die Produktionsmenge einer EO-Anlage konstant zu halten, muß die Reaktionstemperatur bei gleichem Umsatz angehoben werden, wodurch sich der Druck des anfallenden Wasserdampfes erhöht. Die gleichzeitig abnehmende Selektivität führt zu größeren Dampfmengen. Bei üblichen großtechnischen Anlagen fällt häufig zu Betriebsbeginn ein Wasserdampfdruck im Bereich von 30 bar an, mit kontinuierlicher Steigerung über eine Betriebsdauer von 2 Jahren auf einen Wert von etwa 65 bar.

In Abhängigkeit von der zur Verfügung stehenden Energie kann (können) die Dampfturbine(n) eine oder mehrere Arbeitsmaschinen, insbesondere Prozeßpumpen (zum Fördern von Kreiswasser) oder Verdichter (für gasförmige Prozeßströme) und/oder einen oder mehrere Generatoren antreiben.

Der (den) Dampfturbine(n) zugeführte Wasserdampf weist in der Regel einen Druck von 25 bis 70 bar, bevorzugt von 30 bis 65 bar, auf.

In einer besonders bevorzugten Verfahrensvariante wird der bei der Ethylen-Direktoxidation anfallende Wasserdampf in einem Verfahren zur Gewinnung von Monoethylenglykol aus Ethylenoxid durch Hydrolyse, Druckentwässerung, Vakuumentwässerung und anschließender Reindestillation über die Dampfturbine(n) auf den Druck des Sumpfverdampfers der Druckentwässerungskolonne oder des Sumpfverdampfers der ersten Druckentwässerungskolonne einer Kaskade entspannt und der Abdampf der Dampfturbine(n) zur Beheizung der Druckentwässerungskolonne oder der ersten Druckentwässerungskolonne der Kaskade eingesetzt.

Durch ein angepaßtes Design der Glykol-Druckentwässerungsstufen kann die für die Druckentwässerung benötigte Dampfmenge an die in der Ethylendirektoxidation erzeugte Dampfmenge angenähert werden. Damit wird der Fremdbezug von Hochdruckdampf im zeitlichen Mittel deutlich reduziert.

Die Beheizung der Druckentwässerungskolonne oder der ersten Druckentwässerungskolonne der Kaskade erfolgt über einen Sumpfverdampfer und das Kondensat wird zur EO-Reaktionsstufe zurückgeführt.

Gemäß weiterer Ausführungsformen erfolgt die Entspannung über die Dampfturbine(n) auf den Druck eines Dampfnetzes oder auf den Betriebsdruck von Verbrauchern, wie Dampfinjektoren oder Sumpfverdampfer.

Durch das erfindungsgemäße Verfahren kann der spezifische Energieverbrauch einer EO und/oder Monoethylenglykol-Anlage durch einen hohen Wärmeintegrationsgrad gesenkt werden. Die Kolonnen im Glykolverfahren können überwiegend mit kontaminiertem Dampf aus der Druckentwässerung betrieben werden. Ein Fremdbezug von Hochdruckdampf im zeitlichen Mittel wird deutlich reduziert.

Die wirtschaftlich und energetisch günstigste Lösung orientiert sich an den Standortrahmenbedingungen, insbesondere am Niveau der Dampfnetze sowie an den Energiepreisen.

Die Erfindung wird im folgenden anhand einer Zeichnung sowie von Ausführungsbeispielen näher erläutert.

Die Stromdaten in den nachfolgenden Beispielen gelten für den Mittelwert einer Betriebsperiode des Katalysators in der EO-Reaktion.

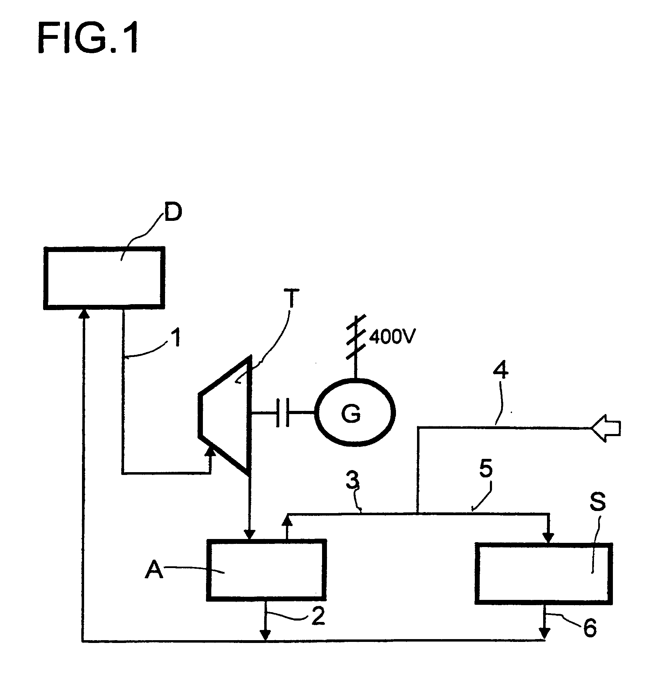

**Figur 1** zeigt schematisch ein Beispiel zur Mehrfachnutzung von Dampf aus der EO-Reaktion in einer Gegendruck-Dampfturbine mit angeschlossenem Generator und Entspannung auf den Gegendruck des Sumpfverdampfers der 1. Stufe der Glykol-Druckentwässerung. Die Stromdaten sind in Tabelle 1 aufgeführt.
Der aus der Dampftrommel D des EO-Reaktors entnommene Sattdampf 1 wird in einer Gegendruck-Dampfturbine T auf einen Druck von 21 bar abs. entspannt. Die Turbine T treibt einen Generator G zur Stromerzeugung (z.B. 400 V) an. Je nach Wirkungsgrad der Turbine T beträgt die Antriebsleistung ca. 2 MW. Die Entspannung in der Turbine T erfolgt ins Naßdampfgebiet, weshalb in einem Abscheider A Kondensat 2 und Sattdampf 3 getrennt werden. Das Kondensat 2 wird in die Dampftrommel D zurück gepumpt. Der Sattdampf 3 wird dem Sumpfverdampfer S der 1. Stufe der Glykol-Druckentwässerung zugeführt. Bei nicht ausreichender Menge wird der Sattdampf 3 mit Netzdampf 4 ergänzt. Das aus dem Sumpfverdampfer S ablaufende Kondensat 6 wird ebenfalls zur Dampftrommel D zurück gepumpt.

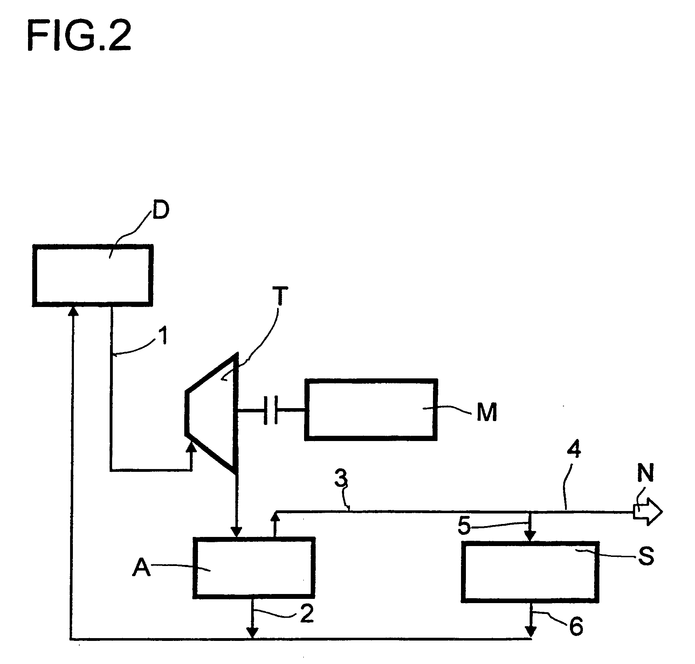

**Figur 2** zeigt ein zweites Beispiel. Die Entspannung erfolgt hier auf einen Gegendruck von 5 bar abs. (Stromdaten siehe Tabelle 2). Mit der Dampfturbine T können eine oder mehrere Arbeitsmaschinen M (Prozesspumpen, Verdichter) mit einer Leistung von ca. 2,7 MW angetrieben werden. Der Sattdampf wird größtenteils dem Sumpfverdampfer S einer Kolonne (Strom 5) zugeführt. Der übrige Sattdampf 4 wird ins Dampfnetz N abgegeben.

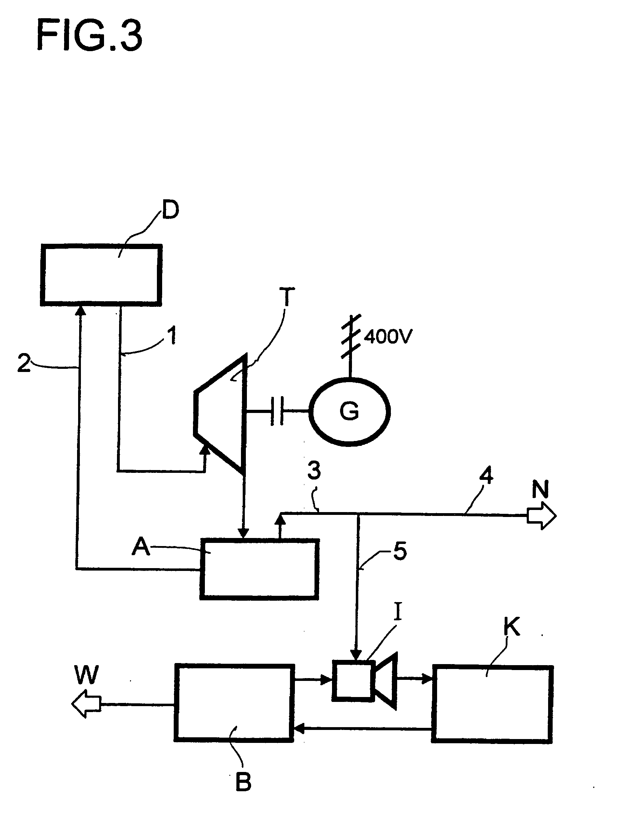

**Figur 3** zeigt ein drittes Beispiel. Die Entspannung erfolgt auf einen Gegendruck von 17 bar abs. (Stromdaten siehe Tabelle 3). Die Dampfturbine T treibt einen Generator G zur Stromerzeugung (z.B. 400 V) mit einer Leistung von ca. 3,8 MW an. Der Sattdampf 3 wird größtenteils ins Dampfnetz N (Strom 4) abgegeben. Mit dem Teilstrom 5 werden ein oder mehrere Dampfinjektoren I betrieben. Im gezeigten Beispiel wird mit Hilfe der Injektoren der Sumpf einer Kolonne K beheizt und gleichzeitig der ablaufende Sumpfstrom durch Erzeugung von Unterdruck in einem nachgeschaltetem Behälter B gekühlt (Verdunstungskühlung). Das Kondensat wird dabei dem Prozesswasser W zugeführt.

**Tabelle 1**

| Strom Nr. 1 | | 1 | 2 | 3 | 4 | 5 | 6 |
|---|---|---|---|---|---|---|---|
| Gesamtstrom | t/h | 52,3 | 2,6 | 49,7 | 9,3 | 59 | 59 |
| Druck | bat _{abs} | 53 | 21 | 21 | 41 | 21 | 21 |
| Temperatur | °C | 268 | 215 | 215 | 400 | 242 | 215 |
| | | g | f | g | g | g | f |
| g: gasförmig f: flüssig | | | | | | | |

**Tabelle 2**

| Strom Nr. 1 | | 1 | 2 | 3 | 4 | 5 | 6 |
|---|---|---|---|---|---|---|---|
| Gesamtstrom | t/h | 31,4 | 2,8 | 28,6 | 12,6 | 16 | 16 |
| Druck | bar _{abs} | 53 | 5 | 5 | 5 | 5 | 5 |
| Temperatur | °C | 268 | 152 | 152 | 152 | 152 | 152 |
| | | g | f | g | g | g | f |
| g: gasförmig f: flüssig | | | | | | | |

**Tabelle 3**

| Strom Nr. 1 | | 1 | 2 | 3 | 4 | 5 | 6 |
|---|---|---|---|---|---|---|---|
| Gesamtstrom | t/h | 83,7 | 4,8 | 78,9 | 63,4 | 15,5 | |
| Druck | bar _{abs} | 53 | 17 | 17 | 17 | 17 | |
| Temperatur | °C | 268 | 204 | 204 | 204 | 204 | |
| | | g | f | g | g | g | |
| g: gasförmig f: flüssig | | | | | | | |

## Patentansprüche

1. Verfahren zur Herstellung von Ethylenoxid durch Direktoxidation von Ethylen mit Luft oder Sauerstoff unter Verwendung von Wasser als Wärmeüberträger zur Abführung der Reaktionswärme, wobei Wasserdampf mit über die Betriebsdauer kontinuierlich ansteigendem Druck entsteht, der anschließend entspannt wird, **dadurch gekennzeichnet, daß** die Entspannung des Wasserdampfes in einer oder mehreren Gegendruck-Dampfturbine(n) T erfolgt.

2. Verfahren nach Anspruch 1, **dadurch gekennzeichnet, daß** die Dampfturbine(n) T eine oder mehrere Arbeitsmaschinen M, insbesondere Prozeßpumpen und Verdichter, und/oder einen oder mehrere Generatoren G antreibt (antreiben).

3. Verfahren nach Anspruch 1 oder 2, **dadurch gekennzeichnet, daß** der (den) Dampfturbine(n) T zugeführte Wasserdampf einen Druck von 25 bis 70 bar, bevorzugt 30 bis 65 bar, aufweist.

4. Verfahren nach einem der Ansprüche 1 bis 3, wonach aus Ethylenoxid durch Hydrolyse, Druckentwässerung, Vakuumentwässerung und anschließender Reindestillation Monoethylenglykol gewonnen wird, und der bei der Ethylen-Direktoxidation anfallende Wasserdampf über die Dampfturbine(n) T auf den Druck des Sumpfverdampfers S der Druckentwässerungskolonne oder des Sumpfverdampfers S der ersten Druckentwässerungskolonne einer Kaskade entspannt wird, und daß der Abdampf der Dampfturbine(n) T zur Beheizung der Druckentwässerungskolonne oder der ersten Druekentwässenmgskolonne der Kaskade eingesetzt wird.

5. Verfahren nach einem der Ansprüche 1 bis 3, **dadurch gekennzeichnet, daß** die Entspannung über die Dampfturbine(n) T auf den Druck eines Dampfnetzes N erfolgt, oder auf den Betriebsdruck von Verbrauchern, insbesondere von Dampfinjektoren oder Sumpfverdampfern.

## Claims

1. A process for preparing ethylene oxide by direct oxidation of ethylene with air or oxygen using water as heat carrier for removing the heat of reaction, with the formation at a pressure rising continuously over the operating period of water vapor that is then expanded, which comprises carrying out the expansion of the water vapor in one or more backpressure steam turbine(s) T.

2. A process as claimed in claim 1, wherein the steam turbine(s) T drives (drive) one or more working machines M, in particular process pumps and compressors, and/or one or more generators G.

3. A process as claimed in claim 1 or 2, wherein water vapor fed to the steam turbine(s) T has a pressure from 25 to 70 bar, preferably from 30 to 65 bar.

4. A process as claimed in any of claims 1 to 3, according to which monoethylene glycol is produced from ethylene oxide by hydrolysis, pressure dehydration, vacuum dehydration and subsequent distillation, and the water vapor arising in the direct oxidation of ethylene is expanded via the steam turbine(s) T to the pressure of the bottoms reboiler S of the pressure dehydration tower or of the bottoms reboiler S of the first pressure dehydration tower of a cascade, and wherein the exhaust steam of the steam turbine(s) T is used for heating the pressure dehydration tower or the first pressure dehydration tower of the cascade.

5. A process as claimed in any of claims 1 to 3, wherein the expansion is carried out via the steam turbine(s) T to the pressure of a steam grid N, or to the operating pressure of consumers, in particular steam injectors or bottoms reboilers.

## Revendications

1. Procédé de préparation d'oxyde d'éthylène au moyen de l'oxydation directe d'éthylène avec de l'air ou de l'oxygène en utilisant de l'eau en tant que fluide caloporteur afin d'évacuer la chaleur de réaction, dans lequel il se forme de le vapeur d'eau avec une pression augmentant continuellement avec la durée de fonctionnement, que l'on détend ensuite, **caractérisé en ce que** l'on réalise la détente de la vapeur d'eau dans une ou plusieurs turbines à vapeur à contre-pression T.

2. Procédé selon la revendication 1, **caractérisé en ce que** la ou les turbines à vapeur T entraînent une ou plusieurs machines M, en particulier des pompes et des condenseurs, et/ou un ou plusieurs générateurs G.

3. Procédé selon la revendication 1 ou 2, **caractérisé en ce que** la vapeur d'eau introduite dans la ou les turbines à vapeur T présente une pression allant de 25 bars à 70 bars, de préférence de 30 bars à 65 bars.

4. Procédé selon l'une quelconque des revendications 1 à 3, dans lequel on obtient du monoéthylèneglycol à partir d'oxyde d'éthylène au moyen d'une hydrolyse, d'une déshydratation sous pression, d'une déshydratation sous vide puis d'une rectification, et dans lequel la vapeur d'eau formée lors de l'oxydation directe d'éthylène est détendue par l'intermédiaire de la ou des turbines T à la pression de l'évaporateur de fond S de la colonne de déshydratation sous pression ou de l'évaporateur de fond S de la première colonne de déshydratation sous pression d'une cascade, et la vapeur de la ou des turbines à vapeur T est mise en oeuvre pour le chauffage de la colonne de déshydratation sous pression ou de la première colonne de déshydratation sous pression de la cascade.

5. Procédé selon l'une quelconque des revendications 1 à 3, **caractérisé en ce que** l'on réalise la détente par l'intermédiaire de la ou des turbines à vapeur T à la pression d'un réseau de vapeur N, ou à la pression de fonctionnement de consommateurs, en particulier d'injecteurs de vapeur ou d'évaporateurs de fond.
